(19) Europäisches Patentamt
European Patent Office
Office européen des brevets

(11) Numéro de publication: **0 358 558 A1**

# (12) DEMANDE DE BREVET EUROPEEN

(21) Numéro de dépôt: **89402406.6**

(22) Date de dépôt: **05.09.89**

(51) Int. Cl.[5]: **A 61 K 31/44**

(30) Priorité: **07.09.88 FR 8811678**

(43) Date de publication de la demande: **14.03.90 Bulletin 90/11**

(84) Etats contractants désignés: **AT BE CH DE FR GB IT LI LU NL SE**

(71) Demandeur: **SYNTHELABO**
**58 rue de la Glacière**
**F-75013 Paris (FR)**

(72) Inventeur: **Bertin, Jean**
**55, rue d'Estienne d'Orves**
**F-92140 Clamart (FR)**

**Frost, Jonathan**
**23, Route de Montjean**
**F-91320 Wissous (FR)**

**Langer, Salomon**
**92, rue Jouffroy**
**F-75017 Paris (FR)**

**Lardenois, Patrick**
**18, rue Varengue**
**F-92340 Bourg la Reine (FR)**

**Wick, Alexander**
**10, Boulevard des Plants**
**F-78860 St Nom La Bretèche (FR)**

(74) Mandataire: **Ludwig, Jacques et al**
**SYNTHELABO Service Brevets 58, rue de la Glacière**
**F-75013 Paris (FR)**

(54) **Utilisation de dérivés de tétrahydro-4,5,6,7 furopyridines pour obtenir des médicaments destinés au traitement de l'obésité.**

(57) Utilisation de dérivés de tétrahydro-4,5,6,7 furopyridines pour obtenir des médicaments destinés au traitement de l'obésité.

EP 0 358 558 A1

## Description

# UTILISATION DE DERIVES DE TETRAHYDRO-4,5,6,7 FUROPYRIDINES POUR OBTENIR DES MEDICAMENTS DESTINES AU TRAITEMENT DE L'OBESITE

La présente invention a pour objet l'utilisation de dérivés de tétrahydro-4,5,6,7 furopyridines pour obtenir des médicaments destinés au traitement de l'obésité.

Les composés utilisables selon l'invention répondent à la formule générale (I)

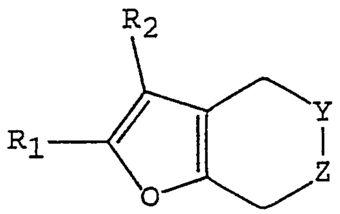

(I)

dans laquelle

$R_1$ représente un groupe phényle, halogénophényle, dihalogénophényle, aminophényle, méthylphényle, éthylphényle, méthoxyphényle, trifluorométhylphényle, phénylphényle, naphtyle ou benzodioxannyle,

$R_2$ représente un atome d'hydrogène ou un groupe méthyle, et

- soit Y représente un groupe $CH_2$ et Z représente un groupe de formule $N-R_3$,
- soit Y représente un groupe de formule $N-R_3$ et Z représente un groupe $CH_2$,

$R_3$ représentant un atome d'hydrogène ou un groupe méthyle, éthyle ou benzyle.

La plupart d'entre eux, ainsi que leur procédé de préparation, sont décrits dans les demandes de brevets européens N°0170549 et 0178201, correspondant aux brevets des Etats Unis d'Amérique N°4621087 et 4661498. Par ailleurs, quatre composés (R1 = H ou F, R2 = H, Y = NH ou $N-CH_2C_6H_5$ et Z = $CH_2$) sont décrits dans Chem. Pharm. Bull. (1977), 25, 1911-1922.

Les composés utilisables selon l'invention peuvent exister à l'état de bases libres ou de sels d'addition à des acides.

Le tableau suivant illustre les structures et points de fusion de quelques composés utilisables selon l'invention.

Tableau

| N° | $R_1$ | $R_2$ | Y | Z | $R_3$ | Sel/ base | F(°C) |
|---|---|---|---|---|---|---|---|
| 1 | $C_6H_5$- | H | $CH_2$ | $NR_3$ | $CH_2C_6H_5$ | HCl | 244-245 |
| 2 | 4-$CH_3O$-$C_6H_4$- | H | $CH_2$ | $NR_3$ | $CH_2C_6H_5$ | HCl | 252-253 |
| 3 | 4-$CH_3$-$C_6H_4$- | H | $CH_2$ | $NR_3$ | $CH_2C_6H_5$ | HCl | 251-253 |
| 4 | 3-$CH_3O$-$C_6H_4$- | H | $CH_2$ | $NR_3$ | $CH_2C_6H_5$ | HCl | 219-221 |
| 5 | 4-Br-$C_6H_4$- | H | $CH_2$ | $NR_3$ | $CH_2C_6H_5$ | HCl | 263-265 |
| 6 | 4-Cl-$C_6H_4$- | H | $CH_2$ | $NR_3$ | $CH_2C_6H_5$ | HCl | 265-267 |
| 7 | 4-F-$C_6H_4$- | H | $CH_2$ | $NR_3$ | $CH_2C_6H_5$ | HCl | 248-250 |
| 8 | $C_6H_5$- | H | $CH_2$ | $NR_3$ | H | HCl | 255-257 |
| 9 | 4-$C_6H_5$-$C_6H_4$- | H | $CH_2$ | $NR_3$ | $CH_2C_6H_5$ | HCl | 270-272 |
| 10 | 4-$CH_3O$-$C_6H_4$- | H | $CH_2$ | $NR_3$ | H | HCl | 226-228 |
| 11 | 4-Br-$C_6H_4$- | H | $CH_2$ | $NR_3$ | H | HCl | 274-275 |
| 12 | 4-$CH_3$-$C_6H_4$- | H | $CH_2$ | $NR_3$ | H | HCl | 250-252 |
| 13 | 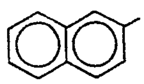 | H | $CH_2$ | $NR_3$ | $CH_2C_6H_5$ | HCl | 249-251 |
| 14 | 3,4-$Cl_2$-$C_6H_3$- | H | $CH_2$ | $NR_3$ | H | HCl | 261-263 |
| 15 | 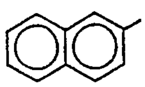 | H | $CH_2$ | $NR_3$ | H | HCl | 265-267 |
| 16 | 4-F-$C_6H_4$- | H | $CH_2$ | $NR_3$ | H | HCl | 254-255 |
| 17 | 3-$CH_3$-$C_6H_4$- | H | $CH_2$ | $NR_3$ | H | HCl | 257-259 |
| 18 | 2-$CH_3$-$C_6H_4$- | H | $CH_2$ | $NR_3$ | H | HCl | 245-247 |
| 19 | 3-$CF_3$-$C_6H_4$- | H | $CH_2$ | $NR_3$ | H | HCl | 273-275 |
| 20 | 2-F-$C_6H_4$- | H | $CH_2$ | $NR_3$ | H | HCl | 284-286 |
| 21 | 3-F-$C_6H_4$- | H | $CH_2$ | $NR_3$ | H | HCl | 258-260 |
| 22 | 4-Cl-$C_6H_4$- | H | $CH_2$ | $NR_3$ | H | HCl | 267-269 |
| 23 | 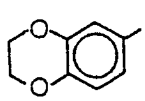 | H | $CH_2$ | $NR_3$ | H | HCl | 260-262 |
| 24 | 4-$C_2H_5$-$C_6H_4$- | H | $CH_2$ | $NR_3$ | H | HCl | 235-237 |
| 25 | 4-$NH_2$-$C_6H_4$- | H | $CH_2$ | $NR_3$ | H | HCl | 258-260 |
| 26 | 3-$C_2H_5$-$C_6H_4$- | H | $CH_2$ | $NR_3$ | H | HCl | 225-226 |
| 27 | 3-$NH_2$-$C_6H_4$- | H | $CH_2$ | $NR_3$ | H | 2HCl | >300 |
| 28 | 4-$CH_3$-$C_6H_4$- | H | $CH_2$ | $NR_3$ | $CH_3$ | HCl | 270-272 |
| 29 | 4-$CH_3$-$C_6H_4$- | H | $CH_2$ | $NR_3$ | $CH_2CH_3$ | HCl | 250-252 |

Tableau (suite)

| N° | $R_1$ | $R_2$ | Y | Z | $R_3$ | Sel/ base | F(°C) |
|---|---|---|---|---|---|---|---|
| 30 | $C_6H_5$- | H | $CH_2$ | $NR_3$ | $CH_3$ | HCl | 263-265 |
| 31 | $C_6H_5$- | H | $CH_2$ | $NR_3$ | $CH_2CH_3$ | HCl | 237-239 |
| 32 | 4-F-$C_6H_4$- | H | $CH_2$ | $NR_3$ | $CH_3$ | HCl | 250-252 |
| 33 | 4-F-$C_6H_4$- | H | $CH_2$ | $NR_3$ | $CH_2CH_3$ | HCl | 263-265 |
| 34 | 4-Br-$C_6H_4$- | H | $CH_2$ | $NR_3$ | $CH_3$ | HCl | 291-293 |
| 35 | 4-Br-$C_6H_4$- | H | $CH_2$ | $NR_3$ | $CH_2CH_3$ | HCl | 262-264 |
| 36 | 4-Cl-$C_6H_4$- | H | $CH_2$ | $NR_3$ | $CH_3$ | HCl | 278-280 |
| 37 | 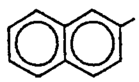 | H | $NR_3$ | $CH_2$ | $CH_2C_6H_5$ | base | 140-142 |
| 38 | 4-$CH_3O$-$C_6H_4$- | H | $NR_3$ | $CH_2$ | $CH_2C_6H_5$ | HCl | 255-257 |
| 39 | $C_6H_5$- | $CH_3$ | $NR_3$ | $CH_2$ | $CH_2C_6H_5$ | HCl | 235-237 |
| 40 | 4-Br-$C_6H_4$- | H | $NR_3$ | $CH_2$ | $CH_2C_6H_5$ | HCl | 271-272 |
| 41 | 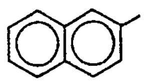 | H | $NR_3$ | $CH_2$ | H | mal. | 190-192(d) |
| 42 | 4-$CH_3$-$C_6H_4$- | H | $NR_3$ | $CH_2$ | $CH_2C_6H_5$ | HCl | 278-280 |
| 43 | 2-$CH_3$-$C_6H_4$- | H | $NR_3$ | $CH_2$ | $CH_2C_6H_5$ | HCl | 258-259 |
| 44 | 4-$CH_3$-$C_6H_4$- | H | $NR_3$ | $CH_2$ | H | HCl | 289-290(d) |
| 45 | 2-$CH_3$-$C_6H_4$- | H | $NR_3$ | $CH_2$ | H | HCl | 244-246 |
| 46 | 4-Cl-$C_6H_4$- | H | $NR_3$ | $CH_2$ | $CH_2C_6H_5$ | HCl | 265-267 |
| 47 | 3-$CH_3$-$C_6H_4$- | H | $NR_3$ | $CH_2$ | H | HCl | 272-275 |
| 48 | 4-$CH_3$-$C_6H_4$- | $CH_3$ | $NR_3$ | $CH_2$ | $CH_2C_6H_5$ | HCl | 232-234 |
| 49 | 4-$CH_3$-$C_6H_4$- | $CH_3$ | $NR_3$ | $CH_2$ | H | HCl | 252-254 |
| 50 | 2-F-$C_6H_4$- | H | $NR_3$ | $CH_2$ | H | HCl | 282-284 |
| 51 | 3-$CF_3$-$C_6H_4$- | H | $NR_3$ | $CH_2$ | H | HCl | 270-272 |
| 52 | 4-$C_2H_5$-$C_6H_4$- | H | $NR_3$ | $CH_2$ | H | HCl | 280-282 |
| 53 | 3-$C_2H_5$-$C_6H_4$- | H | $NR_3$ | $CH_2$ | H | HCl | 264-266 |
| 54 | 4-Cl-$C_6H_4$- | H | $NR_3$ | $CH_2$ | H | HCl | 299-300 |
| 55 | 2-Cl-$C_6H_4$- | H | $NR_3$ | $CH_2$ | H | HCl | 265-267 |
| 56 | 4-$CH_3O$-$C_6H_4$- | H | $NR_3$ | $CH_2$ | H | HCl | 268-269 |
| 57 | 4-Br-$C_6H_4$- | H | $NR_3$ | $CH_2$ | H | HCl | 270-272 |
| 58 | 4-$CH_3O$-$C_6H_4$- | H | $NR_3$ | $CH_2$ | $CH_2H_3$ | HCl | 250-252 |
| 59 | 4-Br-$C_6H_4$- | H | $NR_3$ | $CH_2$ | $CH_3$ | HCl | 256-257 |

Tableau (suite et fin)

| N° | $R_1$ | $R_2$ | Y | Z | $R_3$ | Sel/ base | F(°C) |
|---|---|---|---|---|---|---|---|
| 60 | 4-Cl-$C_6H_4$- | H | $NR_3$ | $CH_2$ | $CH_3$ | HCl | 263-264 |
| 61 | 4-F-$C_6H_4$- | H | $NR_3$ | $CH_2$ | $CH_2C_6H_5$ | HCl | 263-264 |
| 62 | 4-F-$C_6H_4$- | H | $NR_3$ | $CH_2$ | H | HCl | 266-268 |
| 63 | $C_6H_5$- | H | $NR_3$ | $CH_2$ | $CH_2C_6H_5$ | base | 98-99(*) |
| 64 | $C_6H_5$- | H | $NR_3$ | $CH_2$ | H | base | 79-81(*) |

Légende

Dans la colonne "sel/base", HCl désigne le chlorhydrate, 2HCl désigne le dichlorhydrate, et mal. désigne le maléate. Dans la colonne "F(°C)", (d) indique un point de fusion avec décomposition et (*) indique un point de fusion selon Chem. Pharm. Bull (1977), 25, 1911-1922.

L'activité anorexigène des composés a été étudiée chez le rat Sprague-Dawley.

Les animaux, d'un poids de 200 à 250 g, groupés à cinq ou six par cage, sont d'abord maintenus dans un cycle dé 12 h de lumière (7 h-19 h) suivies de 12 h d'obscurité (19 h-7 h).

Avant les essais on les transfère dans des cages individuelles et on les fait jeûner pendant 18 à 20 h, en ne leur fournissant que de l'eau.

Tous les essais commencent le matin entre 9 h et 9 h 30.

On administre aux animaux 5 mg/kg de composé à étudier, par voie intrapéritonéale, les animaux témoins recevant une injection de sérum physiologique.

30 minutes plus tard on leur présente la nourriture habituelle, dans des boîtes de Petri préalablement pesées. Puis, à intervalles de temps déterminés (30 mn, 1 h, 2 h et 4 h) après l'introduction de la nourriture, on en recueille les restes pour les peser, y compris ce qui est tombé à côté de l'écuelle et en dehors de la cage.

Par différence on calcule le poids de nourriture absorbée, et on le rapporte au poids de nourriture absorbée par les animaux de contrôle. On obtient ainsi le pourcentage de nourriture absorbée, qui est donc d'autant plus faible que l'effet anorexigène est plus fort.

Pour la plupart des composés l'effet anorexigène maximal est observé 30 minutes après l'apport de nourriture (soit 1 h après l'administration des composés) ; à cet instant, les pourcentages de nourriture absorbée se situent entre 20 et 75% par rapport aux animaux témoins.

Les résultats des essais montrent que les composés de l'invention sont utilisables pour la préparation de médicaments destinés au traitement de l'obésité.

A cet effet ils peuvent être présentés sous toute forme convenant à l'administration orale ou parentérale, par exemple sous forme de comprimés, dragées, gélules, capsules, solutions ou suspensions buvables ou injectables, en association avec des excipients appropriés.

Ils peuvent être utilisés seuls ou combinés à d'autres substances actives telles que des acides aminés, par exemple le tryptophane ou le hydroxy-5 tryptophane.

La posologie peut aller de 10 à 200 mg de substance active par jour.

**Revendications**

1. Utilisation d'un composé de formule générale (I).

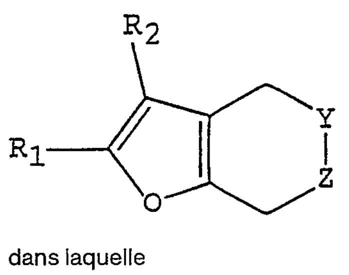

(I)

dans laquelle

$R_1$ représente un groupe phényle, halogénophényle, dihalogénophényle, aminophényle, méthylphényle, éthylphényle, méthoxyphényle, trifluorométhylphényle, phénylphényle, naphtyle ou benzodioxannyle,
$R_2$ représente un atome d'hydrogène ou un groupe méthyle, et
- soit Y représente un groupe $CH_2$ et Z représente un groupe de formule $N-R_3$,
- soit Y représente un groupe de formule $N-R_3$ et Z représente un groupe $CH_2$,
$R_3$ représentant un atome d'hydrogène ou un groupe méthyle, éthyle ou benzyle,
sous forme de base libre ou de sel d'addition à un acide acceptable en pharmacologie,
pour obtenir un médicament destiné au traitement de l'obésité.

2. Utilisation selon la revendication 1, caractérisée en ce que le composé de formule générale (I) est la (bromo-4 phényl)-2 tétrahydro-4,5,6,7 furo[2,3-c]pyridine.

3. Composition pharmaceutique destinée au traitement de l'obésité, obtenue par utilisation d'un composé tel que défini dans la revendication 1.

Office européen des brevets

# RAPPORT DE RECHERCHE EUROPEENNE

Numero de la demande

EP 89 40 2406

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | EP-A-0 178 201 (SYNTHELABO)<br>* Page 11, exemple 8; page 14-16, ligne 13 - page 21, ligne 26 * | 1-3 | A 61 K 31/44 |
| D,A | EP-A-0 170 549 (SYNTHELABO)<br>* Page 11, exemple 11; pages 17-19; page 20, ligne 1 - page 23, ligne 35 * | 1-3 | |
| D,A | CHEM. PHARM. BULL., vol. 25, no. 8, 1977, pages 1911-1922; Y. NAGAI et al.: "Studies on psychotropic agents. II. Synthesis of 1-substituted-3-(p-fluorophenacyl)piperidines and the related compounds"<br>* Page 1920, tableau IV; page 1920, les 2 dernières sections; page 1916, section: "Pharmacology" * | 1,3 | |
| | | | **DOMAINES TECHNIQUES RECHERCHES (Int. Cl.5)** |
| | | | A 61 K |

Le présent rapport a été établi pour toutes les revendications

| Lieu de la recherche | Date d'achèvement de la recherche | Examinateur |
|---|---|---|
| LA HAYE | 22-11-1989 | GOETZ G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)